(19) Europäisches Patentamt
European Patent Office
Office européen des brevets

(11) Publication number: **0 169 375**
**B1**

# (12) EUROPEAN PATENT SPECIFICATION

(45) Date of publication of patent specification: **20.12.89**

(51) Int. Cl.$^4$: **C 07 D 237/14,** C 07 C 109/18

(21) Application number: **85107602.6**

(22) Date of filing: **19.06.85**

## (54) Process for producing 2-tert.-butyl-4,5-dichloro-3(2H)-pyridazinone.

(30) Priority: **23.06.84 JP 129785/84**
**23.06.84 JP 129786/84**

(43) Date of publication of application:
**29.01.86 Bulletin 86/05**

(45) Publication of the grant of the patent:
**20.12.89 Bulletin 89/51**

(84) Designated Contracting States:
**AT BE CH DE FR GB IT LI LU NL SE**

(56) References cited:
**EP-A-0 063 413**

(73) Proprietor: **NISSAN CHEMICAL INDUSTRIES LTD.**
**3-7-1, Kanda Nishiki-cho**
**Chiyoda-ku Tokyo (JP)**

(72) Inventor: **Suzuki, Hideo c/o Central Research Institute**
**Nissan Chemical Ind. Ltd. 722-1, Tsuboi-cho**
**Funabashi-shi Chiba-ken (JP)**
Inventor: **Kawamura, Yasuo c/o Central Research Institute**
**Nissan Chemical Ind. Ltd. 722-1, Tsuboi-cho**
**Funabashi-shi Chiba-ken (JP)**
Inventor: **Ogura, Tomoyuki c/o Central Research Institute**
**Nissan Chemical Ind. Ltd. 722-1, Tsuboi-cho**
**Funabashi-shi Chiba-ken (JP)**

(74) Representative: **Patentanwälte Schaad, Balass & Partner**
**Dufourstrasse 101 Postfach**
**CH-8034 Zürich (CH)**

**EP 0 169 375 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

(a) Field of the Invention

This invention relates to a process for producing 2-tert.-butyl-4,5-dichloro-3(2H)-pyridazinone (hereinafter, referred to as TDCP) which is useful as an intermediate of an active ingredient compound of agricultural drugs, particularly insecticides, acaricides, nematicides, and fungicides.

(b) Description of the Prior Art

Generally, in the synthesis of 2-alkyl-4,5-dichloro-3(2H)-pyridazinone [3] from alkylhydrazine [1] and mucochloric acid [2] starting materials according to the following reaction:

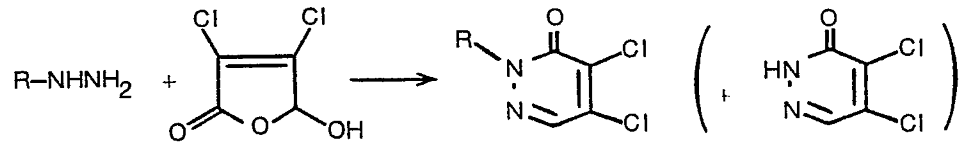

(1) (2) (3) (4)

a good yield is achieved by refluxing the reaction mixture in an aqueous solution of a mineral acid such as hydrochloric acid or sulfuric acid. For example, 2-methyl-4,5-dichloro-3(2H)-pyridazinone can be obtained in a high yield by refluxing a mixture of methylhydrazine and mucochloric acid in an aqueous solution of hydrochloric acid.

According to the above-mentioned method, however, reaction of tert.-butylhydrazine with mucochloric acid provides a low yield of intended TDCP, and a large amount of by-products such as 4,5-dichloro-3(2H)-pyridazinone of the formula [4] above and the like is produced.

On the other hand, in the method described in US—A—4,366,155, TDCP is produced by neutralizing tert.-butylhydrazine hydrochloride with potassium hydroxide in water-ethanol, reacting the tert.-butylhydrazine with mucochloric acid, distilling off the solvent, adding acetic acid and acetic anhydride to replace the solvent therewith, and refluxing the reaction mixture for about 20 hours to conduct reaction again. Also in this method, however, the yield of TDCP has been low.

Thus, there have been problems in conventional methods with respect to separation and purification of TDCP from a large amount of by-products, disposal of by-products, recovery of water-soluble solvents, etc. in addition to practical production of TDCP.

As mentioned above, conventional methods for producing TDCP have not been satisfactory for industrial mass production of TDCP.

Summary of the Invention

An object of the present invention is to provide a process for producing 2-tert.-butyl-4,5-dichloro-3(2H)-pyridazinone in a good yield.

Another object of the invention is to provide a process for producing 2-tert.-butyl-4,5-dichloro-3(2H)-pyridazinone, in which the aimed product can be readily separated from the solvent used and readily purified.

Other objects of the invention will be apparent to those skilled in the art from the description given below.

The present invention provides a process for producing 2-tert.-butyl-4,5-dichloro-3(2H)-pyridazinone, which comprises reacting tert.-butylhydrazine with mucochloric acid characterized by the features of claim 1. Particular embodiments of the invention are set out in the dependent claims 2 and 3.

Detailed Description of the Invention

The process according to the invention is represented by the following reaction schemes:

Reaction (1) tert.$-C_4H_9NHNH_2$ +

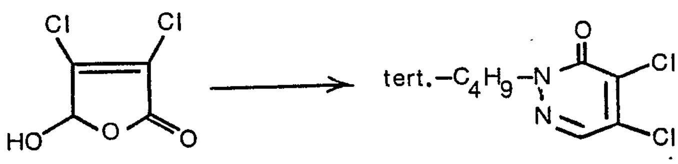

(MCA) (TDCP)

Reaction (2)

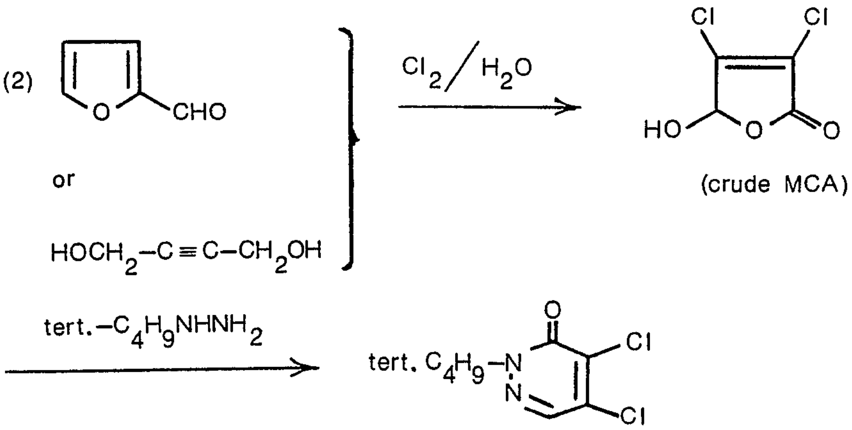


(crude MCA)

(TDCP)

The process represented by reaction (1) is a one-stage method which comprises cyclization reaction of tert.-butylhydrazine with mucochloric acid in a solvent of an aromatic hydrocarbon and/or a halogenated hydrocarbon to give TDCP in a high yield by one stage.

The process according to reaction (2) comprises reacting tert.-butylhydrazine (in a solvent of an aromatic hydrocarbon and/or a halogenated hydrocarbon) with crude mucochloric acid which has been obtained through oxidative chlorination of furfural or 1,4-butynediol, to give TDCP in a high yield.

The present invention will be explained in detail below.

As a tert.-butylhydrazine material to be used in reactions (1) and (2), can be used tert.-butylhydrazine itself or a mineral acid salt (the hydrochloride or the sulfate) thereof. When such mineral acid salt is used, it is preferable to treat the salt previously with a base (such as aqueous NaOH, KOH, $Na_2CO_3$ or $NaHCO_3$ solution), an organic acid alkali salt (such as sodium formate, sodium acetate, potassium propionate and sodium benzoate), which constitutes one of the important features of the invention. It has also been found that TDCP can be obtained in a high yield in the case where a specific organic acid salt of tert.-butyl hydrazine such as the formate or acetate salt thereof is used even without pre-treatment.

As the mucochloric acid to be used as a starting material, commercial available products can be used as they are. Alternatively, crude mucochloric acid obtained through oxidative chlorination of furfural or 1,4-butynediol as shown in the reaction (2) can be used directly. A tert.-butylhydrazine is reacted with preferably about an equal mol of mucochloric acid, and the reaction may be carried out with an excess amount of either one.

The most important feature of the invention resides in using an oil-soluble solvent as illustrated below as a solvent in the cyclization reaction. Namely, particularly preferred oil-soluble solvents include aromatic hydrocarbons represented by benzene, toluene, xylene, ethylbenzene, isopropylbenzene, cymene, tert.-butylbenzene, etc.; and halogenated hydrocarbons represented by chloroform, dichloromethane, carbon tetrachloride, dichloroethane, trichloroethane, tetrachloroethane, dibromomethane, bromoethane, dibromoethane, bromochloromethane, etc. Moreover, can be also used substituted benzenes represented by chlorobenzene, dichlorobenzene, nitrobenzene, benzonitrile, etc.

On the other hand, it is not preferred to use as the solvent alcohols such as methanol, ethanol, propanol, etc.; ethers such as diethylether, tetrahydrofuran, etc.; esters such as methyl acetate, ethyl acetate, etc.; ketones such as acetone, methylethyl ketone, etc.; and amides such as N,N-dimethylformamide (DMF), N,N-dimethylacetamide (DMA), etc., since the yield of the intented TDCP is decreased.

The reaction temperature for the cyclization is desirably in the range of approximately 0°C to 150°C, especially in the range of approximately 10°C to 100°C. It is one of the features of the present invention that addition of the specific organic acid is effective in the cyclization reaction.

Examples of the specific organic acid include formic acid, acetic acid, propionic acid, benzoic acid, p-toluenesulfonic acid etc. Among them, fatty acids such as formic acid, acetic acid, propionic acid and the like are preferred. A satisfactory result can be obtained by the addition of a trace amount of such organic acid when there is no water in the reaction system, and such acid is effective in an amount of about 0.01 time or more the molar amounts of tert.-butylhydrazine or mucochloric acid starting materials.

The process according to the invention is advantageous also in that the end product can be obtained in a higher yield compared with conventional methods and that the end product can be readily purified.

The present invention will be explained specifically by way of the following examples and comparative examples. Incidentally, these examples are merely illustrative and are not to restrict the invention.

## Example 1

To a solution of 5.4 g of 42% aqueous potassium hydroxide solution and 48 g benzene 5.0 g (0.04 mol of tert.-butylhydrazine hydrochloride were added. The resulting mixture was stirred at room temperature

for 15 minutes and after adding 6.8 g (0.04 mol) mucochloric acid, stirred at 40 to 45°C for 5 hours to complete the reaction. Then, water was added to the reaction mixture, and the separated benzene layer was washed successively with 30% aqueous sodium hydroxide solution, 35% hydrochloric acid solution and water, and then dried over anhydrous sodium sulfate. The solvent was distilled off under reduced pressure to give 6.9 g of 2-tert.-butyl-4,5-dichloro-3(2H)-pyridazinone (TDCP) having a melting point of 63—65°C as pale yellow crystals (yield: 78%).

Example 2

To a solution of 4.9 g of 33% aqueous sodium hydroxide solution and 60 g dichloromethane 5.0 g (0.04 mol) tert.-butylhydrazine hydrochloride were added. The resulting mixture was stirred at room temperature for 15 minutes, and after adding 6.8 g (0.04 mol) mucochloric acid, stirred at 40 to 45°C for 6 hours to complete the reaction. Then the following procedures were carried out as in Example 1 to give 66 g of the end product (TDCP) as pale yellow crystals (yield: 75%).

Example 3

To a solution of 4.9 g of 33% aqueous sodium hydroxide solution and 48 g benzene 5.0 g (0.04 mol) tert.-butylhydrazine hydrochloride were added. The resulting mixture was stirred at room temperature for 15 minutes, and after adding 6.8 g (0.04 mol) mucochloric acid, stirred for 30 minutes. Then, 3.6 g of acetic acid was added thereto and the reaction mixture was stirred at 35 to 45°C for 4 hours. After completion of the reaction, the following procedures were carried out as in Example 1 to give 7.4 g of the end product (TDCP) as pale yellow crystals (yield: 84%).

Example 4

To a solution of 3.5 g (0.04 mol) tert.-butylhydrazine and 48 g benzene 6.8 g (0.04 mol) mucochloric acid were added at 10 to 15°C. To the resulting mixture (stirred for 30 minutes) 2.4 g acetic acid were added and then stirred at 35 to 40°C for 4 hours. After completion of the reaction, the following procedures were carried out as in Example 1 to give 7.4 g of the end product TDCP as pale yellow crystals (yield: 84%).

Example 5

The same procedures as in Example 4 were carried out by using 5.9 g (0.04 mol) tert.-butylhydrazine acetate instead of the tert.-butylhydrazine starting material in the example to give 7.7 g of the end product TDCP as pale yellow crystals (yield: 87%).

Example 6

To a solution of 4.9 g of 33% aqueous sodium hydroxide solution and 48 g benzene 5.0 g (0.04 mol) tert.-butylhydrazine hydrochloride were added. The resulting mixture was stirred at room temperature for 15 minutes and after adding 6.8 g (0.04 mol) mucochloric acid stirred for 30 minutes. Then, 3.0 g of propionic acid were added thereto and the reaction mixture was stirred at 35 to 45°C for 4 hours. After completion of the reaction, the following procedures as in Example 1 were repeated to give 7.1 g of the end product TDCP as pale yellow crystals (yield: 80%).

Example 7

To a solution of 4.9 g of 33% aqueous sodium hydroxide solution and 48 g toluene 5.0 g (0.04 mol) tert.-butylhydrazine hydrochloride were added. The resulting mixture was stirred at room temperature for 15 minutes and after adding 6.8 g (0.04 mol) mucochloric acid stirred for 30 minutes. Then, 2.4 g of acetic acid was added thereto and the reaction mixture was stirred at 35 to 45°C for 4 hours. After completion of the reaction, the following procedures as in Example 1 were repeated to give 7.1 g of the end product (TDCP) as pale yellow crystals (yield: 80%).

Example 8

The same procedures as in Example 1 were carried out by using 3.3 g (0.04 mol) of sodium acetate as base and toluene as solvent to give 7.2 g of the end product TDCP (yield: 81%).

Comparative Example 1

To a mixed solution of 24 g acetic acid and 24 g acetic anhydride 3.5 g (0.04 mol) tert.-butylhydrazine and 6.8 g (0.04 mol) mucochloric acid were added at 10 to 15°C and the resulting mixture was stirred at 40 to 45°C for 5 hours. Then, the reaction mixture was concentrated and water and benzene were added. The following procedures were carried out as in Example 1 to give 0.9 g of the end product (TDCP) as pale yellow crystals (yield: 10%).

Comparative Example 2

To a solution of 4.9 g of 33% aqueous sodium hydroxide solution and 48 g ethanol 5.0 g (0.04 mol) tert.-butylhydrazine hydrochloride were added. The resulting mixture was stirred at room temperature for 15 minutes, and after adding 6.8 g (0.04 mol) mucochloric acid stirred at 40 to 45°C for 6 hours. After completion of the reaction, the ethanol was distilled off under reduced pressure and then water was added.

The reaction liquid was extracted with benzene. Then the following procedures were carried out as in Example 1 to give 1.0 g of the end product TDCP as pale yellow crystals (yield: 11%).

Comparative Example 3

The same procedures as in Comparative Example 2 were carried out by replacing the ethanol with ethyl acetate to give 1.1 g of the end product TDCP as pale yellow crystals (yield: 12%).

Example 9

To furfural (9.6 g) and 8% aqueous hydrochloric acid (200 ml) in a 500 ml cylindrical gas-absorption tube chlorine gas was introduced at a rate of 500 ml/min. for 5 hours at 90°C. After completion of the introduction, the reaction liquid was concentrated, extracted with toluene and concentrated again to give 15.1 g of crude crystalline mucochloric acid of 94% in purity.

Then 10.4 g of tert.-butyl hydrazine hydrochloride was dissolved in an aqueous solution of 3.4 g sodium hydroxide in 10 g of water, and the resulting solution was added successively to 100 g toluene and 15.1 g of the crude crystalline mucochloric acid. The resulting reaction mixture was stirred at 40 to 45°C for 5 hours to complete reaction.

The following procedures were carried out as in Example 1 to give 13.7 g of the end product TDCP as crystals.

Example 10

To furfural (9.6 g) and 8% aqueous hydrochloric acid (200 ml) in a 500 ml cylindrical gas-absorption tube chlorine gas was introduced at a rate of 500 ml/min. for 5 hours at 90°C. After completion of the introduction, the reaction liquid was concentrated to give 17.8 g of crude crystals of mucocloric acid.

Then 10.4 g of tert.-butyl hydrazine hydrochloride was dissolved in an aqueous solution of 3.4 g sodium hydroxide in 10 g of water, and the resulting solution was added successively to 100 g benzene and 17.8 g of the crude crystals of mucochloric acid. The resulting reaction mixture was stirred at 40 to 45°C for 5 hours to complete reaction.

The following procedures were carried out as in Example 1 to give 13.1 g of the end product TDCP as crystals.

Example 11

To 1,4-butynediol (8.8 g) and 10% aqueous hydrochloric acid (200 ml) in a 500 ml cylindrical gas-absorption tube chlorine gas was introduced at a rate of 500 ml/min. for 7 hours at 90°C. After completion of the introduction, the reaction liquid was concentrated, extracted with toluene and concentrated again to give 9.2 g of crude crystals of mucochloric acid of 83% in purity.

Then 5.6 g tert.-butyl hydrazine hydrochloride was dissolved in an aqueous solution of 1.8 g sodium hydroxide in 10 g of water, and the resulting solution was added successively to 100 g methylene chloride and 9.2 g of the crude crystals of mucochloric acid. The resulting reaction mixture was stirred at 40 to 45°C for 5 hours to complete reaction.

The following procedures were carried out as in Example 1 to give 7.9 g of the end product TDCP as crystals.

**Claims**

1. A process for producing 2-tert.-butyl-4,5-dichloro-33(2H)-pyridazinone which comprises reacting tert.-butyl-hydrazine with mucochloric acid characterised by carrying out the reaction in a solvent selected from aromatic hydrocarbons, halogenated hydrocarbons and mixtures thereof in the presence of a specific organic acid.

2. The process according to Claim 1, wherein the solvent is selected from benzene, toluene, xylene, ethylbenzene, isopropylbenzene, cymene, tert.-butylbenzene, chloroform, dichloromethane, carbon tetrachloride, dichloroethane, trichloroethane, tetrachloroethane, dibromomethane, bromoethane, dibromoethane, bromochloromethane, and a mixture thereof.

3. The process according to Claim 1 or 2 wherein the organic acid is selected from formic acid, acetic acid, propionic acid and a mixture thereof.

**Patentansprüche**

1. Verfahren zur Herstellung von 2-tert.-Butyl-4,5-dichlor-3(2H)-pyridazinon durch Umsetzung von tert-Butyl-hydrazin mit Mucochlorsäure, dadurch gekennzeichnet, dass man die Umsetzung in einem Lösungsmittel aus der Gruppe von aromatischen Kohlenwasserstoffen, halogenierten Kohlenwasserstoffen und deren Gemischen in Gegenwart einer spezifischen organischen Säure durchführt.

2. Verfahren nach Anspruch 1, dadurch gekennzeichnet, dass man ein Lösungsmittel aus der Gruppe von Benzol, Toluol, Xylol, Ethylbenzol, Isopropylbenzol, Cymol, tert.-Butylbenzol, Chloroform, Dichlormethan, Tetrachlorkohlenstoff, Dichlorethan, Trichlorethan, Tetrachlorethan, Dibrommethan, Bromethan, Dibromethan, Bromchlormethan und ein Gemisch davon verwendet.

3. Verfahren nach Anspruch 1 oder 2, dadurch gekennzeichnet, dass man die organische Säure aus der Gruppe von Ameisensäure, Essigsäure, Propionsäure und ein Gemisch davon verwendet.

**Revendications**

1. Procédé de production de 2-tert.-butyl-4,5-dichloro-3(2H)-pyridazinone, dans lequel on fait réagir de la tert.-butyl-hydrazine avec de l'acide mucochlorique, caractérisé en ce que la réaction est mise en oeuvre dans un solvant choisi parmi les hydrocarbures aromatiques, les hydrocarbures halogénés et leurs mélanges, en présence d'un acide organique spécifique.

2. Procédé selon la revendication 1, dans lequel le solvant est choisi parmi le benzène, le toluène, le xylène, l'éthylbenzène, l'isopropylbenzène, le cymène, le tert.-butylbenzène, le chloroforme, le dichlorométhane, le tétrachlorure de carbone, le dichloroéthane, le trichloroéthane, le tetrachloroéthane, le dibromométhane, le bromoéthane, le dibromoéthane, le bromochlorométhane, et un de leurs mélanges.

3. Procédé selon la revendication 1 ou 2, dans lequel l'acide organique est choisi parmi l'acide formique, l'acide acétique, l'acide propionique et un de leurs mélanges.